# EUROPEAN PATENT SPECIFICATION

(11) **EP 1 361 862 B1**
(45) Date of publication and mention of the grant of the patent: **18.04.2007**
(21) Application number: 02701476.0
(22) Date of filing: 20.02.2002
(51) Int. Cl.: A61K 9/127

(54) **LIPOSOME-MEDIATED DNA ADMINISTRATION**
DURCH LIPOSOME VERMITTELTE DNA VERABREICHUNG
ADMINISTRATION D'ADN INDUITE PAR DES LIPOSOMES

(30) Priority: 22.02.2001 US 270121 P
(43) Date of publication of application: 19.11.2003
(73) Proprietor: OPPERBAS HOLDING B.V., 1102 BR Amsterdam Zuidoost (NL)
(72) Inventor: BARU, Moshe, 37000 Pardes Hana (IL)
(74) Representative: Meyers, Hans-Wilhelm
(86) International application number: PCT/IB2002/000505
(87) International publication number: WO 2002/066013

(56) References cited:
- EP-A- 1 026 253
- WO-A-00/23115
- WO-A-97/10851

## Description

### FIELD OF THE INVENTION

The present invention concerns the use of non-cationic liposomes encapsulating a nucleotide sequence for eliciting an immune response.

### PRIOR ART

The following is a list of references which are intended for better understanding of the background of the present invention.
Gregoriadis, G., Saffie, R, de Souza, J.B, Liposome-mediated DNA vaccination, *FEBS Letters,* **402**:107-110 (1997).
Huang ZM, Yen TSB. Role of the hepatitis B virus posttranscriptional regulatory element in export of intronless transcripts. *Moll Cell Biol* 1995; **15**: 3 864-3 869.
Huang Z, Yen TSB. Hepatitis-B virus-RNA element that facilitates accumulation of surface gene transcripts in the cytoplasm. *J Virol* 1994; **68**: 3193-3199.
Ishii N., Fukushima, J., Kaneko, T., Okada, E., Tani, K., Tanaka, SI., Hamajima, K., Xin KQ., Kawamoto, S., Koff, W, Nishioka, K., Yasuda, T., and Okuda, K in: Cationic liposomes are a strong adjuvant for a DNA vaccine of human immunodeficiency virus type 1, *AIDS Res. Hum. Retroviruses,* **13**(16):1421-8, (1997).
Pavlakis, George, N., and Smyth-Templeton, Nancy, Novel Liposome Complexes for increased Systemic Delivery, WO 9807408, 1998.
Zhou, WZ., Kaneda, Y, Huang, S., Morishita, R., Hoon, D. in: Protective immunization against melanoma by gp 100 DNA-HVJ-liposome vaccine, *Gene Ther.,* **6**(10):1768-73 (1999).

### BACKGROUND OF THE INVENTION

Injection of a DNA sequence encoding specific antigens into a host has shown to induce both humoral and cell-mediated immune responses against the encoded peptide. Liposomes have been used in an attempt to enhance the immune response elicited by antigenic coding DNA. Positively charged (cationic) liposomes encapsulating various DNA constructs administered to a host intramuscularly, intraperitoneally, subcutaneously, intradermally and intranasally induced higher levels of antibody production and delayed-type hypersensitivity (DTH) as compared to the same DNA when administered alone (Ishii *et al.,* 1997). Intramuscular immunization of mice with cationic liposomes encapsulating a DNA plasmid encoding the S region of Hepatitis B surface antigen led to improved humoral and cell-mediated immunity in mice (Gregoriadis *et al.,* 1997). Systemical administration to mice of cationic liposomes comprising DOTAP (dioleoyl trimethylammonium propane) and at least one cholesterol or cholesterol derivative and encapsulating a nucleic acid encoding an expressible protein to mice resulted in enhancement of gene expression, the highest expression being found in the lungs of the animals (Pavlakis *et al.,* 1998). Intramuscular injection of non-cationic liposomes prepared from viral envelopes and containing viral and encapsulating a plasmid DNA encoding a melanoma specific antigen induced autoimmunity against melanoma in a mouse model (Zhou *et al.,* 1999).

WO 97/10851 discloses in vivo gene transfer and expression using non-cationic liposomes comprising a nucleic acid and a polypeptide. This publication does not disclose a humoral immune response in the treated hosts.

### SUMMARY OF THE INVENTION

The present invention is based on findings showing that injection of non-cationic liposomes which are either neutral or have a negative charge not containing peptides and which encapsulate a nucleotide sequence (referred to herein at times as *"DNA* ") intravenously (i.v.) into mice results in expression of the DNA sequence encapsulated within the liposome. The expression occurs mainly in the spleens of the injected mice. Thus, the liposomes when injected systemically, reach the spleen of the host more efficienctly than any other organ of the host resulting in the relatively high expression of the encapsulated sequence in the spleen cells. Moreover, expression of the DNA sequence in the spleen leads to production of antibodies against the expressed protein. In addition, in accordance with the invention, it was shown that spleen cells of the animals injected i.v. with the liposomes of the invention could efficiently be used for the production of monoclonal antibodies against the expressed antigen. Thus, the invention has made it possible to target a desired nucleic acid sequence into the spleen of a host and to elicit in the host an immune response against the peptide encoded by the sequence by encapsulating the desired sequence into non-cationic liposomes which are injected i.v. into the host.

In addition, in accordance with the invention, it was shown that intramuscular (i.m.) administration of non-cationic liposomes which are either neutral or have a negative charge and do not contain viral proteins encapsulating a nucleotide sequence into an animal results in expression of the nucleic acid sequence in the injected animal.

Thus, by its first aspect, the present invention provides a method for eliciting in a host a humoral immune response against a peptide encoded by a nucleotide sequence comprising administrating non-cationic liposomes encapsulating said nucleotide sequence into the host.

The term *"humoral immune response ",* as would be understood by any person versed in the art, concerns any immune response which is not a cellular immune response, including production of antibodies, productions of various growth factors, activation of B-cells, activation of T-helper cells, etc.

The term *"peptide"* should be understood to mean any expression product of the encapsulated nucleic acid.

The term *"nucleic acid sequence"* relates to any nucleotide sequence encoding a peptide including double or single stranded DNA, RNA or a DNA/RNA hybrid, all as detailed below.

The term *"non-cationic liposomes"* concerns liposomes which are either neutral or have a negative charge. The characteristics of such liposomes and examples thereof are described below. By a preferred embodiment the liposomes are larger than 200 nm. By another preferred embodiment these liposomes are multilamellar liposomes.

The term *"host"* in accordance with the invention refers to the recipient of the nucleotide encapsulating liposome composition which may be any vertebrate, preferably a mammal.

In accordance with this aspect of the invention, the non-cationic liposomes encapsulating the nucleotide sequence may be administered to the host by any of the methods known in the art including intramuscularly (i.m.), subcutaneously (s.c.), intradermally (i.d.), intraperitoneally (i.p.), intravenously (i.v.), etc. By a preferred embodiment, the nucleotide sequence encapsulating liposomes are injected i.m. or i.v., most preferably i.v.

By one embodiment of this aspect, the present invention provides a method for the production of antibodies in a host against a peptide encoded by a nucleotide sequence comprising administering non-cationic liposomes encapsulating said nucleotide sequence to the host.

By a preferred embodiment of this aspect of the invention, said non-cationic liposomes encapsulating the nucleotide sequence are administered intravenously (i.v.) or intramuscularly (i.m), preferably, i.v. into the host.

In addition, the present invention provides non-cationic liposomes encapsulating a nucleotide sequence for preparing a composition for eliciting a humoral immune response in a host. By a preferred embodiment, the humoral immune response is the production of antibodies directed against an antigen encoded by said nucleotide sequence.

In accordance with another of its aspects, the present invention provides a method for targeting a nucleic acid sequence administered to a host to the spleen of said host comprising encapsulating said sequence in a non-cationic liposome and administering said nucleotide sequence-encapsulating liposome to the host intraveneously (i.v.).

The term *"targeting"* in accordance with the invention should be understood to mean homing of the liposomes encapsulating the nucleotide sequence administered to the host from the blood to an organ resulting in a high specific activity of the peptide encoded by the encapsulated sequence in the organ as compared to the specific activity of the same peptide in another organ of the host.

The term *"specific activity"* of a peptide should be understood to mean the level of expression of the peptide/mg total protein content in the tissue.

The invention further provides non-cationic liposomes encapsulating a nucleotide sequence for the preparation of an i.v. composition for targeting the nucleic acid into the spleen of a host.

The invention also provides an i.v. composition comprising as a main active ingredient non-cationic liposomes encapsulating a nucleic acid sequence and an i.v. acceptable carrier.

Furthermore, targeting of the liposomes into the spleen of the host in accordance with the present invention results in the production of monoclonal antibodies against a peptide encoded by the encapsulated sequence by the host spleen cells.

Thus, by an additional aspect, the present invention provides a method for the production of monoclonal antibodies against a desired antigen comprising intravenously administering into a host a composition comprising non-cationic liposomes encapsulating a nucleotide sequence encoding said antigen, obtaining spleen cells from said host, preparing a cell line from said spleen cells and obtaining antibodies produced from said cell line.

By another aspect, the present invention provides non-cationic liposomes encapsulating a nucleotide sequence for preparing an i.v. composition for the production of monoclonal antibodies against an antigen encoded by said nucleotide sequence.

### DETAILED DESCRIPTION OF THE INVENTION

In accordance with the present invention, it has been shown that administration to a host of non-cationic liposomes which are neutral or have a negative charge and do not contain a viral peptide and which encapsulate a nucleotide sequence encoding a desired peptide, results in expression of the nucleotide sequence in the administered host and eventually elicits a humoral immune against the expression product of the sequence response. The non-cationic liposomes used according to the invention may be produced commercially and, due to their non-toxic nature, may be administered in relatively high concentrations to the host.

Targeting of a sequence or a peptide to a specific organ usually requires use of a targeting or homing moiety which has a high affinity to a target organ and thus delivers the sequence or peptide to the specific organ. In accordance with the invention, administration of non cationic liposomes comprising a desired nucleotide sequence into a host results in expression of the encoded sequence mainly in the spleen of the host without any need for coupling of the liposome to an organ-specific homing or targeting ligand such as a peptide, a monoclonal antibody, sugar, glycolipid or protein. Targeting of the desired sequence into the spleen results in expression of the nucleotide with a high specific activity of the peptide in the target spleen cells as compared to the specific activity of the encoded peptide in the cells of other host organs. In addition, such specific targeting of the liposomes into the spleen and expression of the nucleic acid sequence within the spleen cells results in production of antibodies against the peptide encoded by the injected sequence. In view of the fact that the non-cationic liposomes are non toxic, it is possible to inject a large amount of the DNA encapsulating liposomes so as to increase antibody production against antigens encoded by the injected sequence in the host. In addition, as a result of antibody production in the spleen against the injected DNA sequence product, such spleens may be used in the preparation of monoclonal antibodies against the injected DNA sequence.

The nucleotide sequence of the invention is an antigen-encoding sequence such as a double or single-stranded DNA molecule which can be inserted into a gene expression vector, preferably a viral or bacterial plasmid gene expression vector, an RNA molecule or a molecule consisting of both ribonucleotides and deoxynucleotides (RNA/DNA hybrid). The insertion of the nucleotide sequence into the expression vector may be by any of the methods known in the art. The gene expression vector may also include any additional regulatory sequences required for expression of the peptide encoded by the nucleotide sequence such as promoter sequences, recognition sequences, secretion sequences and enhancers, all of which are easily chosen by a person versed in the art.

In accordance with the invention, the nucleotide sequence may encode for more than one antigen, to which a host immune reaction will be elicited. In some cases, the additional antigen may be an immunostimulatory sequence capable of further enhancing the immune reaction against the desired antigen.

The nucleotide sequence of the invention may be obtained by any of the methods known in the art and may be isolated from any polynucleotide sequence of any organism or synthesized chemically.

The polynucleic acid is typically encapsulated within the aqueous interior of the liposome.

The liposomes used in accordance with the invention are non-cationic liposomes which are either neutral or have a negative charge and do not contain viral proteins. Although the liposomes may be of a variety of sizes, in accordance with the invention, they preferably have a diameter larger than 200nm in size. Most types of liposomes belong to either one of the following three types: multilamellar vesicles (MLV), small unilamellar vesicles (SUV) and large unilamellar vesicles (LUV). MLVs typically form spontaneously upon hydration of dried phospholipids. SUVs may be formed from MLVs by sonication and, unlike the multilayered, "onion-like" structure of MLVs, are single layered. SUVs are small with a high surface-to-volume ratio and thus have the lowest capture volume of aqueous space per weight of lipid.

As distinct from SUVs, LUVs have a large aqueous compartment and a single, or at times a few, lipid layers.

In accordance with the invention any of the above liposomes may be used, prefered liposomes being multilamellar liposomes.

The liposomes may be comprised of a variety of lipids, including phospholipids, glycolipids, cholesterol, etc. Preferably, the phospholipids constitute a major component in the liposomal membranes. Preferred phospholipids are lecitines (also known as phosphatidyl-cholines), which are mixtures of diglyceride of stearic, palmitic and oleic acids linked to the choline ester of phosphoric acid. Lecitines are found in and are obtainable from animals and plants. Preferred sources of lecitines are eggs, soybeans, animal tissues such as brain, heart, and the like. Lecitines can also be produced synthetically. As will no doubt be appreciated by the artisan, the source of the phospholipid is immaterial to the present invention and any phospholipid will likely be suitable.

Examples of specific phosphatides are phosphtidyl glycerol, phosphtidyl ethanol amine, L-α-(distearoyl) lecitin, L-α-(diapalmitoyl) lecitin, L-α-phosphatide acid, L-α-(dilauroyl)-phosphatidic acid, L-α-(dimyristoyl) phosphatidic acid, L-α-(dioleoyl) phosphatidic acid, DL-α-(dipalmitoyl) phosphatidic acid, L-α-(distearoyl) phosphatidic acid, and the various types of L-α -phosphatidylcholines prepared from brain, liver, egg yolk, heart, soybean and the like, or synthetically, and salts thereof. Other suitable modifications include the controlled peroxidation of the fatty acyl residue cross-linkers in the phosphatidylcholines (PC) and the zwitterionic amphiphates which form micelles by themselves or when mixed with the PCs such as alkyl analogues of PC.

In addition to phospholipids, the liposomes may also comprise various other lipophilic or amphophilic molecules. The composition of the lipid membrane may be tailored for a variety of specific uses, either to obtain certain stability, size distribution, etc.

The lipids and the other lipophilic or amphophilic molecules constitute together about 1-30% (W/V) of the composition's volume, preferably about 10%. In addition to the composition's ingredients, mentioned above, the composition may also comprise various preservatives and antioxidants.

The compositions are preferably dehydrated-rehydrated vesicles (DRV). DRVs are prepared by rehydration of a dehydrated composition which, upon addition of water, spontaneously forms the composition of the invention. DRVs may be prepared in a number of ways. By one exemplary way, a mixture is first prepared consisting of SUVs and the polynucleic acid molecule. The mixture is then lyophilized to a water content of less than 2%. The reconstitution is typically a multi-stage procedure wherein the first step is a low volume rehydration, i.e., rehydration with a volume of an aqueous solution (which may be water or preferably an aqueous solution comprising salts (e.g. NaCl) or other solutes (e.g. sugar) to yield isotonicity with body fluids, i.e. an osmolarity of about 300 mOsm), equal to about a third or less than the final water volume. Typically, as noted above, the compositions for use in the invention comprise, on a weight per volume basis, about 10% lipids and a low volume of the aqueous solution in the first hydration step. The second hydration step is typically addition of water to yield a concentration of lipids of about 30% (w/v). Then aqueous solution is further added gradually to yield the final volume.

The liposome composition prepared for administration to a host is preferably adjusted to a physiologically acceptable salt concentration (such as, for example, 150 mM NaCl).

In another exemplary way, DRVs can be prepared by mixing the ingredients of the composition with a tertiary alcohol (e.g. tert-butanol) -in-water solution, with the alcohol concentration being in the range of about 10-30%. The concentration of the alcohol typically is correlated with the hydrophobicity of the lipid ingredients, with higher hydrophobicity requiring a higher alcohol concentration. The mixture is then lyophilized since alcohol is more volatile than water, lyophilization is more rapid than that achieved with a solution comprising water alone. The rehydration is performed in a similar manner to that described above.

The manner of preparation of liposomes and their tailoring to suit a specific need is generally known to the artisan and is outside the scope of the present writing. Non-limiting examples of methods for the preparation of liposomes such as those mentioned above are reverse-phase evaporation (RPE) (Kameda *et al, supra*) or the protein-cochleate technique (Gould-Fagerite *et al, supra*).

The desired antigen encoded by the encapsulated nucleotide sequence and against which an immune response is elicited may be any peptide or protein, such as for example human factor VIII. Wherein the humoral immune response results in the production of antibodies (as is the preferred case), these antibodies elicited against the peptide encoded by the encapsulated nucleotide sequence may be of any of the antibody classes IgG, IgM, IgA, IgE or IgD. The immune response elicited against the expressed nucleic acid product may be measured by any of the methods known in the art such as ELISA, RIA etc.

The dosage of the expression vector comprising the nucleotide sequence encapsulated within the liposome will vary depending on the desired response by the host and the antigenic nucleotide used. The dosage must be sufficient to cause expression of the antigenic peptide encoded by the nucleotide sequence at a level which will be sufficient to induce the desired immune reaction.

In accordance with the invention, monoclonal antibodies (MoAbs) may be prepared using any technique which provides for the production of antibody molecules by continuous cell lines in culture. These include but are not limited to the hybridoma technique originally described by Koehler and Milstein *(Nature* **256**:495-497, (1975)), the human B-cell hybridoma technique (Kosbor *et al., Immunol. Today* **4**:72, (1983); *Cote et al., Proc. Natl. Acad. Sci.* **80**:2026-2030, (1983)) and the EBV-hybridoma technique (Cole, *et al., Mol. Cell Biol.* **62**:109-120, (1984)). As seen in the examples below, use of spleen cells from spleens of hosts injected i.v. with the nucleotide encapsulating liposomes of the invention results in production of MoAbs against the antigen encoded by the injected nucleotide sequence.

The invention will now be illustrated by the following non-limiting examples.

### EXAMPLES

### Materials and Methods

### 1. Mice used for injection of liposomes were:

In Examples 1 and 2: hemophilic mice (Bi, L., *et al.* (1995) Nature Genetics 10:119-121) were used. In Example 3: BALB/c mice were used. In Example 4: C57/bl mice were used.

### 2. Expression vectors:

### Factor VIII expression vector pCI-FVIII-PRE (Examples 1 and 2):

This plasmid was constructed in two stages:
1) pCI-FVIII is the pCI plasmid (Promega) containing human FVIII cDNA, which encodes FVIII protein with a deletion of 894 amino acids of its B domain (amino acids746-1639).
2) pCI-FVIII-PRE was constructed by the insertion of the post-transcriptional regulatory element (PRE) of hepatitis B virus (Huang *et al.,* 1994, 1995) into Not I and Sal I sites in the pCI-FVIII plasmid.

### Luciferase expression vector pCI-luc-PRE (Examples 3 and 4):

This plasmid was constructed in two stages:
1) Construction of pCI-luc by the insertion of the firefly luciferase gene (Xho I-Xba I fragment of pGL3-basic (Promega, Madison, WI, USA)) into Xho I and Xba I sites in pCI plasmid (Promega).
2) Construction of pCI-luc-PRE by the insertion of the post-transcriptional regulatory element (PRE) of hepatitis B virus (Huang *et al.,* 1994, 1995) into Not I and Sal I sites in the pCI-luc plasmid.

The plasmid pSec Tag2 (Invitrogen, USA) contains the CMV promoter, Murine 1g Kappa-Chain V-J2-C signal peptide, multiple cloning site, polyhistidine tag cDNA, c-myc epitope cDNA and BGH polyadenylation sequence.

The plasmid pSATag2 (Invitrogen) contains CMV promoter, Murine Ig Kappa-Chain V-J2-C signal peptide, Prostate Specific Antigen (PSA) cDNA, polyhistidine tag cDNA, c-myc epitope cDNA and BGH polyadenylation sequence.

### 3. Liposome preparation (all examples):

Liposomes were prepared as follow: To produce 1 g of liposomes composed of EPC (Lipoid KG, Ludwigshafen, Germany) and DMPG (Sygena AG, Liestal, Switzerland), a tert-butanol solution of EPC and DMPG was prepared by dissolving 0.9086 g EPC and 0.0914 g DMPG in 10 ml tert-butanol (Riedel-de Haen, Seelze, Germany) and 1 ml H₂O. The mixture was mixed with plasmid DNA, dissolved in H₂O to a ratio of 100:1 (w/w) lipids to DNA and then freeze dried. Before use, the dry lipids and DNA was hydrated in two stages. First, H₂O was added to 30% of DNA (v/w) **[should this be w/v?]** and the mixture was shaken until a homogenous solution was obtained. The solution was then adjusted to 150 mM NaCl using 200 mM NaCl and filtered through 15 µm filter to eliminate large aggregates from the solution. For making liposomes composed of EPC only, similar methods were used without adding DMPG. Liposomes comprising EPC:DOPE:DOTAP [DOTP **[should this be DOTAP?]** = 1,2-Dioleoyloxy-3-(trimethyl-ammonium) propane (Avanti Polar Lipids Inc.)] (4:2:1 molar ratio) were prepared as described above.

### [These abbreviations should be defined].

To obtain reduced sized liposomes, the liposome solution was filtered through 0.2 and 0.1 µm polycarbonate filters in a LipoFast Large apparatus (Avestin inc., Ottawa, ON, Canada) to form liposomes of 150-200 µm.

### 4. Measurement of anti-FVIII antibodies (Example 1):

Measurement of anti-FVIII antibodies in mouse plasma was performed using the Bethesda assay as described in Practical Haematology, Sir John V. Dacie and S.M. Lewis, Seventh Edition, (1991) p312-313. The titer of antibodies is in Bethesda units (BU).

### 5. Measurement of luciferase activity (Examples 3 and 4):

Extraction of luciferase from organs was performed as follows: Collected organs were quickly frozen in liquid nitrogen and pulverized into a fine powder. One hundred milligram of tissue powders were homogenized with cell culture lysis reagent (500 µl each, Promega). After a 30-min incubation at room temperature, the samples were frozen and thawed three times and briefly centrifuged to remove cell debris. The supernatant was collected and stored at -78°C until used. The luciferase activity in 20 µl lysate were determined by the addition of 100 µl of luciferase assay reagent (Promega) using a luminometer (TD-20/20, Turner Designs, USA). Relative light units were converted to picogram of luciferase according to a calibration curve based on recombinant luciferase (Promega) as a standard. Protein concentration of the lysate was also determined with the BCA protein assay kit (Pierce, Rockfords, IL, USA).

### Measurement the titer of mouse anti-PSA and anti-6 x Histidine antibodies:

The titer of mouse anti-PSA and anti-6 x Histidine antibodies were measured by specific enzyme-linked immunoabsorbent assay (ELISA). PSA protein, or 6 x histidine peptide (synthesized by Sigma, UK) were used to coat high binding plates (Coming, USA). Mouse serum samples were then added and incubated for 1 hour at 22-25°C. Bound mouse antibodies were detected with alkaline phosphatase conjugated anti-mouse IgG monoclonal antibody (Sigma, USA) and p-nitrophenyl phosphate (Sigma, USA). Commercial monoclonal antibodies against 6 x Histidine (Invitrogen, USA) and PSA (Santa Cruz Biotechnology, USA) were used to plot a calibration curve.

### Example 1

BALB/c and hemophilic mice were injected with 500 µl of liposomes containing the human factor-FVIII expression vector pcI-FVIII-PRE. A second group of mice was injected with 10 units of recombinant (FVIII (rFVIII) (Kogenate-FS, Bager), and 500 µl of liposomes containing the luciferase expression vector only were injected to a group of mice as control. The mice were bled twenty-two days post-injection and the titer of antibodies against human FVIII was measured. The results indicated that a high titer (200-500 BU/ml) of anti-human FVIII antibodies was produced in the mice receiving the encapsulated FVIII. A second injection of liposomes containing the FVIII Control groups of mice that received injections of uncapsulated rFVIII protein or liposome-encapsulated luciferase expression vector, did not produce anti-human FVIII antibodies. A second injection of liposomes containing FVIII expression vector increased the antibody titer.

As can be seen in Table 1 below, the production of anti-FVIII antibodies following i.v. injection of liposome-encapsulated DNA was high as compared to undetectable production of anti-FVIII antibodies in mice injected with recombinant human factor VIII alone or with control liposomes encapsulating the luciferase expression vector.

**Table 1**

| **Production of anti-FVIII antibodies following injection of liposome-encapsulated DNA into mice** | |
|---|---|
| **Injected material** | **Titer of anti-human factor VIII antibodies (Bµ/ml)** |
| Human factor VIII expression vector in liposomes | 200 |
| Recombinant human factor VIII | 0 |
| Luciferase expression vector liposomes Untreated | 0 |

### Example 2 Production of monoclonal antibodies against human FVIII

Factor VIII expression vector (pCI-FVIII-PRE) was encapsulated in EPC-DMPG liposomes (700 nm) as described above and intravenously injected (500 µl) into hemophilic mice. Twenty-one days latter, the mice received a second injection of pCI-FVIII-PRE encapsulated in EPC-DMPG liposomes. Forty-two days after the first injection, the mice were bled and the titer of anti-FVIII antibodies was measured by a Bethesda assay and ELISA. Mice with a high titer of anti-FVIII antibodies (>100 Bethesda Units) were selected for isolation of spleen cells and hybridoma fusing. Fifty colonies of hybridoma cells were isolated. ELISA assay indicated that each of the colonies secreted monoclonal antibodies directed against human FVIII.

### Example 3

EPC-DMPG liposomes having a diameter of 800 or 160 nm and encapsulating the luciferase expression vector were administered to fed or starved mice. Luciferase activity was determined as described above in various organs obtained from the administered mice one day following administration of the luciferase encapsulating liposomes.

As seen in Table 2, as detected by luciferase activity, the highest level of expression observed was in the spleen of fed mice after administration of the large EPC-DPMG liposomes.

**Table 2**

| | **Large EPC-DMPG Lip. (800 nm) Starved mice (n=4)** | **Large EPC-DMPG Lip. (800 nm) Starved mice (n=4)** | **Small EPC-DMPG Lip. (160 nm) Starved mice (n=4)** | **Small EPC-DMPG Lip. (160 nm) Starved mice (n=4)** |
|---|---|---|---|---|
| Liver | 0.03 ± 0.01 | 0.03 ± 0.01 | 0.99 ± 0.59 | 0.11 ± 0.05 |
| Lungs | 0.53 ± 0.23 | 2.34 ± 0.34 | 0.42 ± 0.22 | 0.65 ± 0.03 |
| Heart | 0.71 ± 0.68 | 0.51 ± 0.15 | 0.27 ± 0.19 | 0.17 ± 0.22 |
| Spleen | 2.11 ± 0.94 | 4.34 ± 1.5 | 0.26 ± 0.11 | 0.49 ± 0.19 |

### Example 4

Various kinds of liposomes encapsulating the luciferase expression vector prepared as above were injected intramuscularly (i.m.) to mice. One day after injection of the liposomes, luciferase activity was determined and compared to luciferase activity in mice injected with the naked luciferase DNA vector only.

As seen in Table 3 below, high luciferase activity was seen in mice injected with the luciferase expression vector encapsulated in non-cationic EPC and EPC:DMPG liposomes in accordance with the invention as compared to a very low activity of luciferase activity in mice injected with the cationic EPC:DOPE:DOTAP liposomes or naked DNA.

**Table 3**

| **Injected Material** | **Luciferase fg/mg protein at 1 day post-injection (average), n** |
|---|---|
| DNA in EPC liposomes | 2769 (n = 3) |
| DNA in EPC: DMPG liposomes | 1531 (n = 3) |
| DNA in EPC:DOPE:DOTAP liposomes | 21 (n = 3) |
| Naked DNA | 499 (n = 9) |

### Example 5

### Titer of antibodies following injection of liposome-encapsulated expression vectors or naked expression vectors into mice:

Four groups of 5 mice (Bal/c) were injected i.m. or i.v. with the naked or liposome-encapsulated expression vectors pSecTag or pPSATag, and a fifth control group was injected with PBS. A second dose was injected at 25 days post injection and at 51 days post injection. The mice were bled and the titers of antibodies against PSA and 6x histidine were measured according to a calibration curve of commercial monoclonal antibodies.

As shown in Table 4, the titer of antibodies against PSA was about 110 times higher in mice injected with the liposome-encapsulated expression vectors than in mice injected i.m. with the naked expression vectors. In addition, the titers of antibodies against 6 x histidine were significantly higher in mice injected with liposome-encapsulated expression vectors of both types (pSecTags and pPSATags) than in mice injected with the naked expression vectors. Since the difference in antibody titer was demonstrated using two expression vectors and two different antigens, it can be concluded that vaccination of mice by the liposome-encapsulated DNA of the invention is significantly more efficient than vaccination by naked DNA injection.

In addition, the results indicate that mice, injected with liposome-encapsulated pSecTags, produced antibodies against 6 x histidine. Mice injected with the liposome-encapsulatd pPSATags expression vector produced antibodies against PSA and also antibodies against 6 x histidine. This indicates that the presence of the tags at the c-terminus of a fusion protein does not interfere with production of antibodies against the protein at the n-terminus. Therefore, the presence of anti-tag antibodies indicates an induction of humoral immune response against the fused protein and a high probability of antibody production against the n-terminus protein. This finding can be used as a method to identify mice producing antibodies against any unknown protein whose cDNA is cloned upstream to the cDNA of tags in an appropriate expression vector that is encapsulate in liposomes and injected into the mice.

**Table 4**

| **Treatment** | **Titer of anti PSA antibodies (µg IgG/ml)*** | **Titer of anti 6 x histidine antibodies (µg IgG/ml)*** |
|---|---|---|
| IV injection of pSecTags encapsulated in liposomes | 0 | 27 |
| IM injection of naked pSecTags | 0 | 0 |
| IV injection of pPSATags encapsulated in liposomes | 88 | 38 |
| IM injection of naked pPSATags | 0.8 | 18 |
| PBS | 0 | 0 |

| | | |
|---|---|---|
| * In pool serum samples of 5 mice | | |

## Claims

1. Use of non-cationic liposomes not containing a viral protein and encapsulating a nucleotide sequence for the manufacturing of a composition for eliciting a humoral immune response in a host against a peptide encoded by the nucleotide sequence.

2. The use according to Claim 1, wherein the liposomes are administered intravenously.

3. The use according to Claim 1, wherein the liposomes are administered intramuscularly.

4. The use according to Claim 1, wherein the liposomes have a diameter of at least 200 nm.

5. The use according to Claim 1, wherein the nucleotide is expressed in spleen cells of the host.

6. A method for producing antibodies against a peptide comprising:
(a) administering to a host non-cationic liposomes not containing a viral protein and encapsulating a nucleotide sequence encoding for the peptide; and
(b) collecting the antibodies.

7. The method according to Claim 6 wherein the nucleotide sequence includes a tag sequence encoding a tag product, and wherein the method further comprises determining whether the host produces antibodies against the tag product, production of antibodies against the tag product being indicative of a host producing antibodies against the peptide.

8. The method according to Claim 7 wherein the tag sequence is polyhistidine tag cDNA or c-myc epitope cDNA.

9. The method according to Claim 6, wherein the liposomes are administered intravenously.

10. The method according to Claim 6, wherein the liposomes are administered intramuscularly.

11. The method according to Claim 6, wherein the liposomes have a diameter of at least 200 nm.

12. The method according to Claim 6, wherein the nucleotide is expressed in spleen cells of the host.

13. A method for producing a. cell line producing monoclonal antibodies against a peptide comprising:
(a) administering to a host non-cationic liposomes not containing a viral protein and encapsulating a nucleotide sequence encoding for the peptide so as to express the nucleotide sequence in cells of the host and to yield host cells producing antibodies against the peptide;
(b) obtaining cells producing the antibody; and
(c) forming a hybridoma with the obtained cells.

14. The method according to Claim 13, wherein the liposomes are administered intravenously.

15. The method according to Claim 13, wherein the liposomes are administered intramuscularly.

16. The method according to Claim 13, wherein the liposomes have a diameter of at least 200 nm.

17. The method according to Claim 13, wherein the nucleotide is expressed in spleen cells of the host.

## Patentansprüche

1. Verwendung von nichtkationischen Liposomen, die kein virales Protein enthalten und eine Nucleotidsequenz einkapseln, zur Herstellung einer Zusammensetzung zum Hervorrufen einer humoralen Immunantwort bei einem Wirt gegen ein Peptid, das von der Nucleotidsequenz codiert wird.

2. Verwendung gemäß Anspruch 1, wobei die Liposomen intravenös verabreicht werden.

3. Verwendung gemäß Anspruch 1, wobei die Liposomen intramuskulär verabreicht werden.

4. Verwendung gemäß Anspruch 1, wobei die Liposomen einen Durchmesser von wenigstens 200 nm haben.

5. Verwendung gemäß Anspruch 1, wobei das Nucleotid in Milzzellen des Wirts exprimiert wird.

6. Verfahren zur Herstellung von Antikörpern gegen ein Peptid, umfassend:
(a) Verabreichen von nichtkationischen Liposomen, die kein virales Protein enthalten und eine für das Peptid codierende Nucleotidsequenz einkapseln, an einen Wirt; und
(b) Gewinnen der Antikörper.

7. Verfahren gemäß Anspruch 6, wobei die Nucleotidsequenz eine Tag-Sequenz beinhaltet, die ein Tag-Produkt codiert, und wobei das Verfahren weiterhin Folgendes umfasst: Bestimmen, ob der Wirt Antikörper gegen das Tag-Produkt produziert, wobei die Produktion von Antikörpern gegen das Tag-Produkt einen Wirt anzeigt, der Antikörper gegen das Peptid produziert.

8. Verfahren gemäß Anspruch 7, wobei es sich bei der Tag-Sequenz um Polyhistidin-Tag-cDNA oder c-myc-Epitop-cDNA handelt.

9. Verfahren gemäß Anspruch 6, wobei die Liposomen intravenös verabreicht werden.

10. Verfahren gemäß Anspruch 6, wobei die Liposomen intramuskulär verabreicht werden.

11. Verfahren gemäß Anspruch 6, wobei die Liposomen einen Durchmesser von wenigstens 200 nm haben.

12. Verfahren gemäß Anspruch 6, wobei das Nucleotid in Milzzellen des Wirts exprimiert wird.

13. Verfahren zur Herstellung einer Zelllinie, die monoklonale Antikörper gegen ein Peptid produziert, umfassend:
(a) Verabreichen von nichtkationischen Liposomen, die kein virales Protein enthalten und eine für das Peptid codierende Nucleotidsequenz einkapseln, an einen Wirt, so dass die Nucleotidsequenz in Zellen des Wirts exprimiert wird und Wirtszellen entstehen, die Antikörper gegen das Peptid produzieren;
(b) Gewinnen von Zellen, die den Antikörper produzieren; und
(c) Bilden eines Hybridoms mit den erhaltenen Zellen.

14. Verfahren gemäß Anspruch 13, wobei die Liposomen intravenös verabreicht werden.

15. Verfahren gemäß Anspruch 13, wobei die Liposomen intramuskulär verabreicht werden.

16. Verfahren gemäß Anspruch 13, wobei die Liposomen einen Durchmesser von wenigstens 200 nm haben.

17. Verfahren gemäß Anspruch 13, wobei das Nucleotid in Milzzellen des Wirts exprimiert wird.

## Revendications

1. Utilisation de liposomes non cationiques ne contenant pas de protéine virale et encapsulant une séquence de nucléotides pour la fabrication d'une composition destinée à induire, chez un hôte, une réponse immunitaire humorale contre un peptide codé par la séquence de nucléotides.

2. Utilisation selon la revendication 1, dans laquelle les liposomes sont administrés par voie intraveineuse.

3. Utilisation selon la revendication 1, dans laquelle les liposomes sont administrés par voie intramusculaire.

4. Utilisation selon la revendication 1, dans laquelle les liposomes ont un diamètre d'au moins 200 nm.

5. Utilisation selon la revendication 1, dans laquelle le nucléotide est exprimé dans les cellules spléniques de l'hôte.

6. Procédé de production d'anticorps dirigés contre un peptide comprenant:
(a) l'administration à un hôte de liposomes non cationiques ne contenant pas de protéine virale et encapsulant une séquence de nucléotides codant pour le peptide ; et
(b) le prélèvement des anticorps.

7. Procédé selon la revendication 6, dans lequel la séquence de nucléotides comprend une séquence marqueur codant pour un produit marqueur et dans lequel le procédé comprend en outre l'étape consistant à déterminer si l'hôte produit des anticorps dirigés contre le produit marqueur, la production d'anticorps dirigés contre le produit marqueur indiquant qu'un hôte produit des anticorps dirigés contre le peptide.

8. Procédé selon la revendication 7, dans lequel la séquence marqueur est de l'ADNc du marqueur polyhistidine ou de l'ADNc de l'épitope c-myc.

9. Procédé selon la revendication 6, dans lequel les liposomes sont administrés par voie intraveineuse.

10. Procédé selon la revendication 6, dans lequel les liposomes sont administrés par voie intramusculaire.

11. Procédé selon la revendication 6, dans lequel les liposomes ont un diamètre d'au moins 200 nm.

12. Procédé selon la revendication 6, dans lequel le nucléotide est exprimé dans les cellules spléniques de l'hôte.

13. Procédé de production d'une lignée cellulaire produisant des anticorps monoclonaux dirigés contre un peptide comprenant :
(a) l'administration à un hôte de liposomes non cationiques ne contenant pas de protéine virale et encapsulant une séquence de nucléotides codant pour le peptide, de façon que la séquence de nucléotides s'exprime dans les cellules de l'hôte et de façon à fournir des cellules hôtes produisant des anticorps dirigés contre le peptide ;
(b) l'obtention de cellules produisant l'anticorps ; et
(c) la formation d'un hybridome avec les cellules obtenues.

14. Procédé selon la revendication 13, dans lequel les liposomes sont administrés par voie intraveineuse.

15. Procédé selon la revendication 13, dans lequel les liposomes sont administrés par voie intramusculaire.

16. Procédé selon la revendication 13, dans lequel les liposomes ont un diamètre d'au moins 200 nm.

17. Procédé selon la revendication 13, dans lequel le nucléotide est exprimé dans les cellules spléniques de l'hôte.
